# EUROPEAN PATENT APPLICATION

(11) **EP 2 756 795 A1**
(43) Date of publication of application: **23.07.2014**
(21) Application number: 13152195.7
(22) Date of filing: 22.01.2013
(51) Int. Cl.: A61B 1/267

(54) **Intubation device**

(71) Applicant: Swengg Co., Model Town, Sialkot (PK)
(72) Inventor: Iqbal, Javaid, Sialkot Cantt. (PK)
(74) Representative: Patentanwälte Westphal, Mussgnug & Partner

(57) **Abstract**

The invention relates to a blade (5) for intubation devices (1), the blade (5) comprising an elongated tongue (9) with a proximal end and a distal end, a guiding part (7) extending perpendicularly from an upper surface of the tongue (9) and from the proximal end in direction of the distal end, whereas the tongue (9) is made of metal or a combination of metal and plastics and the guiding part (7) is made of plastics or a combination of plastics and metal.

## Description

The present invention relates to intubation devices also known as laryngoscopes and particularly blades for such intubation devices. Laryngoscopes are used in medicine e.g. for laryngoscopy and tracheal intubation of patients.

Laryngoscopy is a medical procedure that is used to obtain a view of the vocal folds and the glottis. Laryngoscopy may be performed to facilitate tracheal intubation during general anesthesia or cardiopulmonary resuscitation or for procedures on the larynx or other parts of the upper tracheobronchial tree.

Direct laryngoscopy is usually carried out with the patient lying on his or her back; the laryngoscope is inserted into the mouth on the right side and flipped to the left to trap and move the tongue out of the line of sight, and, depending on the type of blade used, inserted either anterior or posterior to the epiglottis and then lifted with an upwards and forward motion ("away from you and towards the roof"). This move makes a view of the glottis possible. There are at least ten different types of laryngoscope used for this procedure, each of which has a specialized use for the otolaryngologist and medical speechpathologist. This procedure is most often employed in direct diagnostic laryngoscopy with biopsy. It is extremely uncomfortable and is not typically performed on conscious patients, or on patients with an intact gag reflex.

Tracheal intubation, usually simply referred to as intubation, is the placement of a flexible plastic tube into the trachea (windpipe) to maintain an open airway or to serve as a conduit through which to administer certain drugs. It is frequently performed in critically injured, ill or anesthetized patients to facilitate ventilation of the lungs, including mechanical ventilation, and to prevent the possibility of asphyxiation or airway obstruction.

The most widely used route is orotracheal, in which an endotracheal tube is passed through the mouth and vocal apparatus into the trachea.

Because it is an invasive and extremely uncomfortable medical procedure, intubation is usually performed after administration of general anesthesia and a neuromuscular-blocking drug. It can however be performed in the awake patient with local or topical anesthesia, or in an emergency without any anesthesia at all. Intubation is normally facilitated by using a conventional laryngoscope, flexible fiberoptic bronchoscope or video laryngoscope to identify the glottis, though other devices and techniques are available.

After the trachea has been intubated, a balloon cuff is typically inflated just above the far end of the tube to help secure it in place, to prevent leakage of respiratory gases, and to protect the tracheobronchial tree from receiving undesirable material such as stomach acid. The tube is then secured to the face or neck and connected to a anesthesia breathing circuit, bag valve mask device, or a mechanical ventilator.

Tracheal intubations usually involve the use of a laryngoscope. The modern conventional laryngoscope consists of a handle containing batteries that power a light and a set of interchangeable blades, which are either straight or curved. This device is designed to allow the laryngoscopist to directly view the larynx.

The decision to use a straight or curved laryngoscope blade depends partly on the specific anatomical features of the airway, and partly on the personal experience and preference of the laryngoscopist. The Macintosh blade is the most widely used curved laryngoscope blade, while the Miller blade is the most popular style of straight blade. Both Miller and Macintosh laryngoscope blades are available in sizes 0 (infant) through 4 (large adult). There are many other styles of straight and curved blades, with accessories such as mirrors for enlarging the field of view and even ports for the administration of oxygen. These specialty blades are primarily designed for use by anesthetists and otolaryngologists, most commonly in the operating room.

Presently laryngoscope blades are produced of metal, i.e. stainless steel, titan or alloys usually used in medicine.

Due to hygienic reasons in the present time there is a tendency towards one way medicine products, thus avoiding extensive and costly cleaning procedures like sterilization. Furthermore the risks of those cleaning procedures, i.e. remaining bacteria or other infectants, are avoided, as well. Referring again to laryngoscopes there are different approaches to provide for the advantages of one-way instruments and facing the emerging cost pressure at the same time.

As described above the laryngoscopes are basically two part instruments consisting of a handle, usually carrying a light source and the necessary energy source, i.e. batteries and a set of interchangeable blades.

A first approach thus uses common stainless steel laryngoscope blades only once with a multi-use handle. Thus there is no need for cleaning the blades which are inserted in the human trachea while the handle, which is carrying the light source may be used again.

A second approach uses complete single use laryngoscopes, including the handle and the blade. The laryngoscopes therefore are completely made of plastics thus drastically lowering the costs for the single use instruments.

A third approach applies single-use plastic blades with a multi-use handle. This approach once again lowers the costs for the single-use part by reducing material expenses. Furthermore this approach allows the use of high quality handles with included power source.

As the blades according to the first approach do not meet the cost pressure on the producers and the users of such instruments the second and third approach sufficiently reduce costs but have some drawbacks in application of the blades.

One major drawback of these approaches is a lack of stability of the plastic blades. As the general shape and size of the blades is predetermined by the kind of blade (Miller, Macintosh, etc.) and the size necessary for the present patient, the freedom of design is quite low. Furthermore the available plastic materials are also restricted to those permitted to medicinal use.

Particularly the area connecting the handle and the blade, also referred to as proximal end or also heel, needs to bear the main force applied by the laryngoscopist for lifting the epiglottis. Furthermore the distal (front) part of the blade, also referred to as the tongue, needs to lift the epiglottis in a controllable and reproducible manner for the laryngoscopist. As the crossection of the blade at the proximal end is quite big and thus the necessary stability may be provided by use of enough plastic material and stabilization. At the distal end the crossection of the tongue needs to be quite small in order to allow the laryngoscopist to view the vocal apparatus and to introduce. However the blades at the same time need to be stable enough to transmit the force to lift the epiglottis from the proximal end to the distal end of the blade particularly to the tip of the tongue.

The present plastic blades are not able to provide enough stability in this area resulting in bending and torsion of the tongue of the blade. The bending and torsion cause irreproducible results leading to serious risks like fatal delays or injury.

A further disadvantage results from the used material. Since the lower surface of the blades is not only for repelling the patients tongue but also as a sliding surface it is necessary to provide a smooth surface allowing a lubricating film to develop. However hydrophobic plastic materials are repelling water thus avoiding the development of a continuous film of water providing for lubrication.

As the blades are only used once and thrown away afterwards it is more and more necessary to reduce the costs for the blades.

It is a task of the present invention to provide blades for intubation devices avoiding the above drawbacks while lowering the manufacturing costs at the same time.

This problem is solved by a blade for a intubation device according to claim 1 and a intubation device with such blades according to claim 14.

A blade for intubation devices according to the present invention comprises an elongated tongue with a proximal end and a distal end, a guiding part also referred to as web extending perpendicularly from an upper surface of the tongue and from the proximal end in direction of the distal end, wherein the tongue is made of metal or metal and plastics and the guiding part is made of plastics or plastics and metal.

By using the above-mentioned hybrid approach one may reach both, sufficient stability of the blade and cost reduction to meet the present needs of the market. A tongue of either metal or metal and plastics and a web of either plastics or plastics and metal guarantee to fulfill the above requirements. The plastic parts provide enough cost reduction while the metal parts provide the stability of the blade.

In a preferred embodiment the tongue of the blade is made of metal.

A metal tongue on the one hand provides for the above-mentioned stability while a lower surface of the metal tongue allows a lubricating film to develop during use of the intubation device. As described above it is advantageous, if the lower surface of the tongue is made of metal because metal better than plastics allows the development of a lubricating film while providing for stability at the same time.

In another embodiment the blade comprises a stiffening extending perpendicularly from the upper surface of the tongue. The stiffening may for example be made of metal. For example the stiffening may be part of the guiding part and for example may be an insert of the guiding part.

A stiffening, especially when designed as an insert, does not need to have a high quality surface because it has not direct tissue contact. Furthermore a stiffening extending perpendicularly from the tongue on an upper surface provides stiffness against bending and torsion of the tongue.

In a preferred embodiment the stiffening is arranged within or next to the guiding part. Depending on the design of the blade it is preferable to either arrange the stiffening within the guiding part or next to it. An arrangement within the guiding part, which in this case is preferably made of plastics, allows the above-mentioned reduced requirements for surface quality and in addition also allows e.g. spot welding or other joining techniques which in medical engineering are not sufficient to provide enough surface quality.

In this embodiment the stiffening is not necessarily affixed to the tongue. A coupling may be achieved e.g. by a push fit of the tongue to the guiding part which may or may not include the stiffening.

In another preferred embodiment of the blade according to the present invention the guiding part is injection molded.

Injection molding is a well developed technique for production of plastic parts and allows the insertion of metal parts either during the molding process or by providing a holding fixture for inserting the metal parts. The metal parts may be fixed to the plastic parts e.g. by a push fit or by a snap on connection. Security of the afore-mentioned connections may be provided by e.g. bolting, screwing or gluing.

The stiffening in another embodiment extends at least from the proximal end of the tongue and covers at least 80 %, preferably 90 % of the length of the tongue. In a further embodiment the stiffening exceeds the proximal end of the tongue.

It is preferable, if the stiffening covers at least 80 % and preferably 90 % of the length of the tongue. By applying a stiffening over this length it is possible to transfer the force from the handle of the blade to the distal end of the blade.

In a preferred embodiment a height of the stiffening decreases continuously, linearly or partially linearly.

If a stiffening is used it is necessary that the height of this stiffening decreases starting from the proximal end of the tongue to allow the laryngoscopist to view the glottis of the patient.

In a further embodiment the stiffening is affixed to the tongue. A fixture may be accomplished for example by welding or gluing. Another option is to integrally form the tongue and the stiffening e.g. by press bending.

The afore-mentioned connection is well approved and allows cost efficient and secure connecting of the parts.

In an advantageous embodiment the tongue and the guiding part are fixed together with a push-fit. A push fit has the advantage that it may easily be combined with e.g. a snap in connection for locking the parts together.

The plastic parts and the metal parts may be molded together to achieve a secure connection of the parts. Furthermore injection molding of metal and plastic components is a well developed method for connecting parts of different materials.

In further embodiment the blade comprises a coupling at its proximal end which is either fixed to or integrally formed with the guiding part of the blade.

A coupling may be used for fixation of the blade to a handle with which it may be used. The coupling may either be fixed to the guiding part or may be integrally formed with the guiding part, e.g. by injection molding. If the coupling and the guiding part are two separate parts a fixation may be accomplished by e.g. screwing or bolting.

An intubation device according to the present invention comprises a blade as described above.

The invention disclosed will hereinafter be described in more detail and with reference to exemplary embodiments depicted in the accompanying drawings. The drawings show curved, so called Macintosh blades, however the present invention may also be applied to other sorts curved or straight blades, e.g. Miller or Parrott blades. The drawings show:
- Fig. 1: a perspective view of a intubation device according to present invention,
- Fig. 2.: a perspective view of a first embodiment of the blade used in the intubation device of Fig. 1,
- Fig.3: a perspective view of a second embodiment of the blade used in the intubation device of Fig. 1, and
- Fig. 4: a perspective view of a third embodiment of the blade used in the intubation device of Fig. 1.

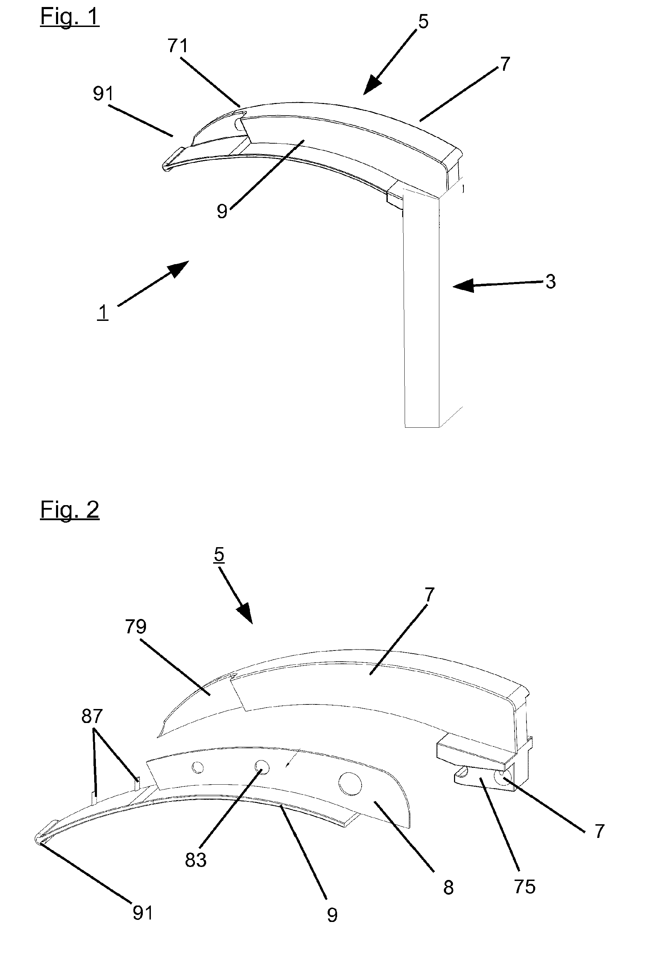

Figure 1 shows a perspective view of an intubation device 1 also referred to as laryngoscope.

The intubation device 1 comprises a blade 5 which with its proximal end is connected to a handle 3. During tracheal intubation the laryngoscopist holds the handle 3 and transfers the motion of the handle 3 to the blade 5. A lower elongated part of the blade is of convex shape and extends from the proximal end of the blade 5 to a distal end called tip 91, which in the present embodiment is of rounded shape to minimize the risk of injuries. The tongue 9 of the present embodiment is made of stainless steel. Extending perpendicularly form the tongue 9 the blade 5 comprises a guiding part 7 for e.g. insertion of a tube in the trachea of the patient. The guiding part is for example made of plastics.

Figure 2 shows an exploded view of a blade 5 consisting of two parts, the guiding part 7 and the blade 9.

The guiding part 7 in the embodiment of figure 2 at its proximal end holds the coupling 75, hereinafter also referred to as heel. The heel 75 is adapted for coupling the guiding part 7 to the handle 3, which is not depicted in the present view.

A height of the guiding part 7 continuously decreases in direction of the distal end. The distal part furthermore forms a recess 73 in lateral direction thus providing space for an outlet e.g. of an optical fiber connected to a light source, a light source or a video optic (both not shown).

The tongue 9 comprises a stiffening 8 which is extending in length direction of the tongue 9 and perpendicularly to its surface. The stiffening exceeds the tongue in proximal direction and covers approximately 75% of the length of the tongue 9. In distal direction of the stiffening 8 the tongue 9 further comprises securing pins 87 which also extend perpendicularly from the surface of the tongue 9.

The stiffening 8 is dislocated from the border of the tongue to a line lying on the surface of the tongue 9 about one quarter of the width of the tongue 9. The securing pins 87 are arranged at the border of the tongue 9.

The stiffening further has openings 83. The securing pins 87 and the openings 83 both serve as connections to the guiding part 7. The tongue 9 and the stiffening 8 may be welded or glued together and are both made of metal to provide sufficient stability of the blade 5. A connection between the tongue 9 with the stiffening 8 and the guiding part 7 is accomplished by injection molding. The tongue 9 and the stiffening 8 are thus molded together with the guiding part 7 which engages the openings 83 and the securing pins 87. Both the securing pins 87 and the stiffening 8 are thus arranged within the guiding part 7.

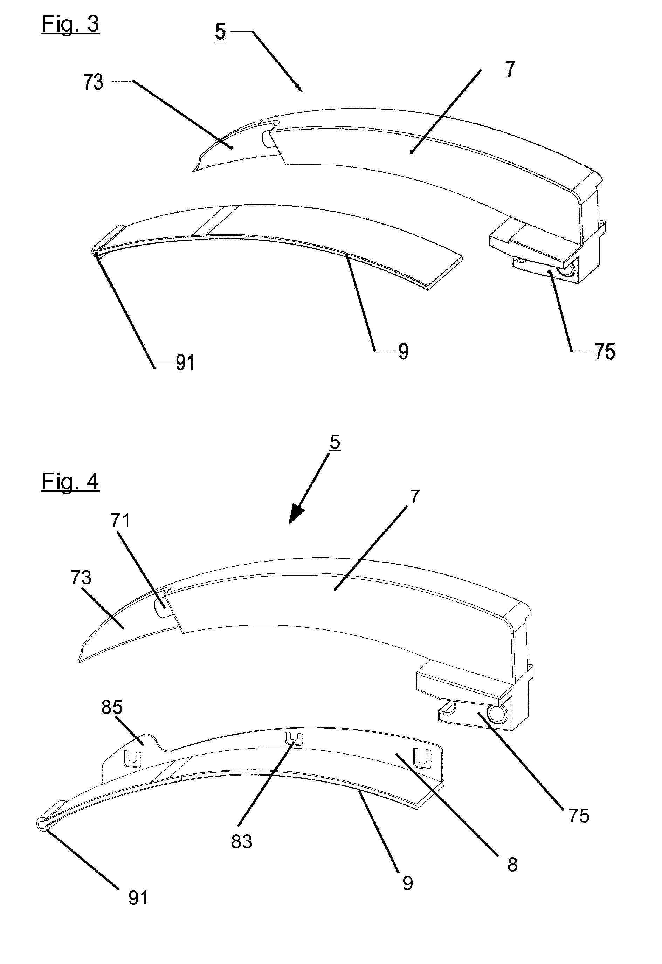

Figure 3 shows a second embodiment of a blade 5 which may be used with the intubation device 1 of figure 1. Figure 3 depicts an exploded view of the blade 5 that consists of the above-mentioned two main parts, the tongue 9 and the guiding part 7.

In the embodiment of figure 3 there is no stiffening connected to the tongue 9. In the present embodiment the tongue 9 and the guiding part 7 may be coupled to each other by a push fit. The guiding part therefore exhibits a groove which is adapted to receive the tongue 9 which is of strip-like shape.

Figure 4 shows an exploded view of a third embodiment of a blade 5 which may be used in the intubation device 1 of figure 1.

The blade according to figure 4 also comprises the two main parts, the tongue 9 including a stiffening 8 and the guiding part 7. At the recess 73 of the guiding part 7 an optical fiber 71 or a light source or a video optic 71 exits the guiding part 7 at a front side directed to the proximal end of the blade 5. The optical fiber 71 is enclosed in the guiding part 7 and may for example be connected via heel 75 to a light source or video optic which may be located within the handle 3 (not depicted in this figure).

The tongue 9 including the stiffening 8, which has u-shaped openings 83 may be connected via a push fit which e.g. may be secured by snap in connections formed via the central nose located between the arms or the u-shaped opening 83.

Generally it is also possible to apply the main idea of the present invention to blades 5 of both, curved and straight shape. Furthermore it is possible to leave the rounded tip 91 raw with only smoothened edges.

Even if not described in connection with the above embodiments the blade may be formed of a metal-plastics-compound, which can save further costs in production.

The above description only shows exemplary embodiments which shall not narrow the scope of the present invention.

### List of reference signs

- 1: intubation device
- 3: handle
- 5: blade
- 7: guiding part
- 8: stiffening
- 9: tongue

- 71: optical fiber
- 73: recess
- 75: heel / coupling

- 83: opening
- 87: securing pin

- 91: tip

## Claims

1. Blade (5) for intubation devices (1), the blade (5) comprising
an elongated tongue (9) with a proximal end and a distal end,
a guiding part (7) extending perpendicularly from an upper surface of the tongue (9) and from the proximal end in direction of the distal end,
**characterized in that**
the tongue (9) is made of metal or a combination of metal and plastics and
the guiding part (7) is made of plastics or a combination of plastics and metal.

2. The blade (5) according to claim 1,
**characterized in that**
the guiding part (7) is made of a combination of plastics and metal.

3. The blade (5) according to one of the preceding claims,
**characterized in that**
the tongue (9) is made of metal.

4. The blade (5) according to one of the preceding claims,
**characterized in that**
the blade (5) comprises a preferably metal stiffening (8) extending perpendicularly from the upper surface of the tongue (9).

5. The blade (5) according to one of the preceding claims,
**characterized in that**
the stiffening (8) is arranged within or next to the guiding part (7).

6. The blade (5) according to one of the preceding claims,
**characterized in that**
the guiding part (7) is injection molded.

7. The blade (5) according to claim 4,
**characterized in**
the stiffening (8) extending from the proximal end of the tongue (9) and covering preferably 90% of the length of the tongue (5).

8. The blade (5) according to one of claims 4 - 7
**characterized in that**
the stiffening (8) exceeds the proximal end of the tongue (9).

9. The blade (5) according to one of claims 4 to 8,
**characterized in that**
a height of the stiffening (8) decreases continuously, linearly or partially linearly.

10. The blade according to one of claims 3 - 6,
**characterized in that**
the stiffening (8) is affixed to the tongue (9).

11. The blade (5) according to claim 7,
**characterized in that**
the stiffening (8) and the tongue (9) are welded or glued together.

12. The blade (5) according to one of claims 3 - 6,
**characterized in that**
the stiffening (9) and the tongue (9) are formed integrally.

13. The blade (5) according to claim 9,
**characterized in that**
the blade (5) and the stiffening (8) are a press-bent part.

14. The blade (5) according to one of the preceding claims,
**characterized in that**
the tongue (9) and the guiding part (7) are fixed together with a push-fit.

15. The blade (5) according to one of claims 1 - 12,
**characterized in that**
the plastic parts and the metal parts are molded together.

16. The blade (5) according to one of the preceding claims,
**characterized in that**
the blade (5) comprises a coupling (75) at its proximal end which is either fixed to or integrally formed with the guiding part (7) of the blade (5).

17. The blade (5) according to one of the preceding claims,
**characterized in that**
the tongue (9) and the guiding part (7) are fixed together with glue.

18. The blade (5) according to one of the preceding claims,
**characterized in that**
the tongue (9) and the guiding part (7) are fixed together with a bolt or a screw.

19. The blade (5) according to one of claims 4 - 18,
**characterized in that**
the stiffening (9) is part of the guiding part (7).

20. Intubation device (1) comprising a blade (5) according to one of the preceding claims.
